# EUROPEAN PATENT APPLICATION

(11) **EP 2 412 388 A1**
(43) Date of publication of application: **01.02.2012**
(21) Application number: 11175082.4
(22) Date of filing: 22.07.2011
(51) Int. Cl.: A61L 2/08

(54) **Equipment for the treatment of materials with ionizing radiations**

(30) Priority: 28.07.2010 IT TO20100650
(71) Applicant: GILARDONI S.p.A., 20124 Milano (IT)
(72) Inventor: Gilardoni, Marco, 23826 Mondello Lario- LC (IT); Ascani Orsini, Andrea, 23826 Mondello Lario - LC (IT)
(74) Representative: Di Gennaro, Sergio

(57) **Abstract**

Equipment for the treatment of materials by means of ionizing radiation, comprising an irradiation chamber (3) inside which the products to be irradiated can be positioned, and in which an extractable drum (5) provided with a plurality of compartments (51) for containers of material to be irradiated can be positioned.

Said drum (5) is able to turn itself in the chamber, so to enable a uniform exposure of all the compartments (51) to the radiations, said rotation of the drum substantially occurring about its longitudinal axis, in order to expose its contents in a homogeneous way to a beam which impinges perpendicular to the axis of rotation itself.

## Description

The present invention refers to an equipment for the treatment of materials by means of ionizing radiation.

In particular, the present invention can be used for irradiating all those substances needing such a treatment. For example, in the medical field, biological substances are irradiated, such as blood, plasma, organs used for transplants and other substances for oncological curative purposes, or sterilization purposes.

In particular, sacs of plasma are exposed to radiations, before their contents are injected in a patient for a transfusion.

The equipments of the known type normally comprise an emission source of ionizing radiations, adapted to send a predetermined quantity of such radiations, generated toward an irradiation chamber inside which the materials to be treated are positioned. Normally, a processing unit is adapted to control the intensity of the beams and their emission time.

A main requirement for the efficiency of such equipments is that the materials are irradiated in an efficient and uniform way. So an important role is the fact that the power of irradiating beam which are emitted be able to reach the materials and their spatial distribution.

Furthermore, the products which undergo the treatment can be of quantity and shape very different one from the other, and therefore the versatility of the equipment is also an important property for such equipments.

The present invention provides an equipment for the treatment of materials by irradiation, able to adapt itself to different needs, according to the type of treatment to be made.

In particular, the equipment has an irradiation chamber in which a module with a rotary drum can be inserted or released, according to the type of material to be treated. Furthermore, the irradiation source or the beam-emitting source and the said chamber are movable one with respect to the other, in order to regulate the size of the beams which hit the irradiation chamber.

An aspect of the present invention refers to an equipment for the treatment of materials by means of radiation, having the properties of the annexed claim 1.

Further additional properties are contained in the dependent annexed claims.

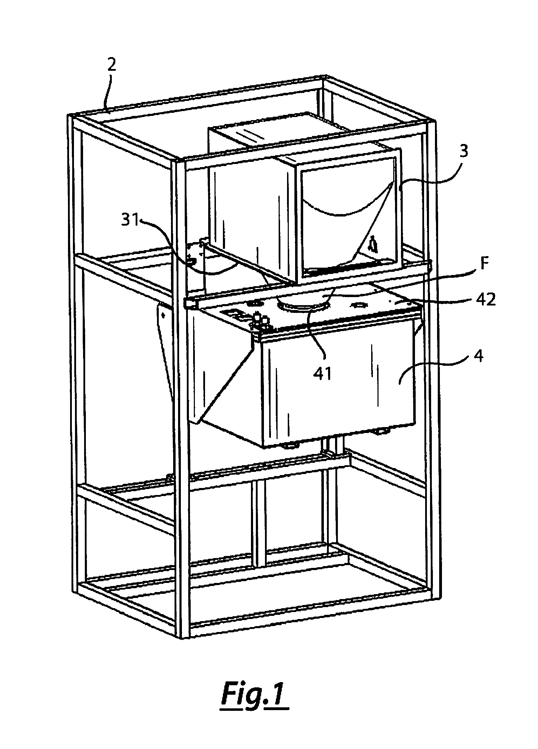

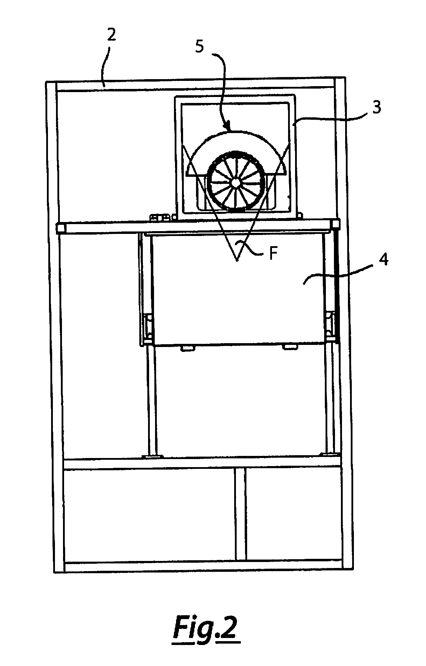

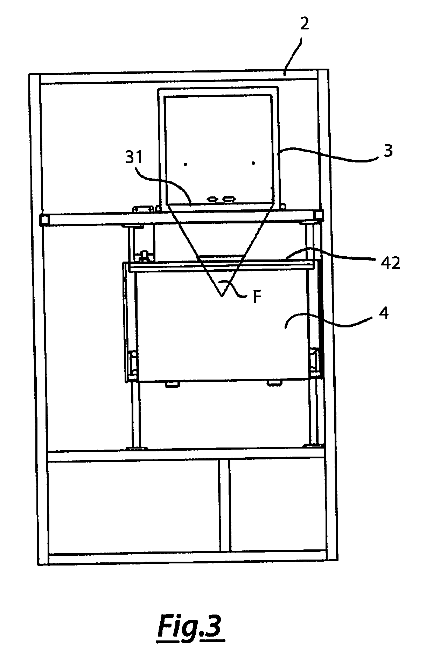

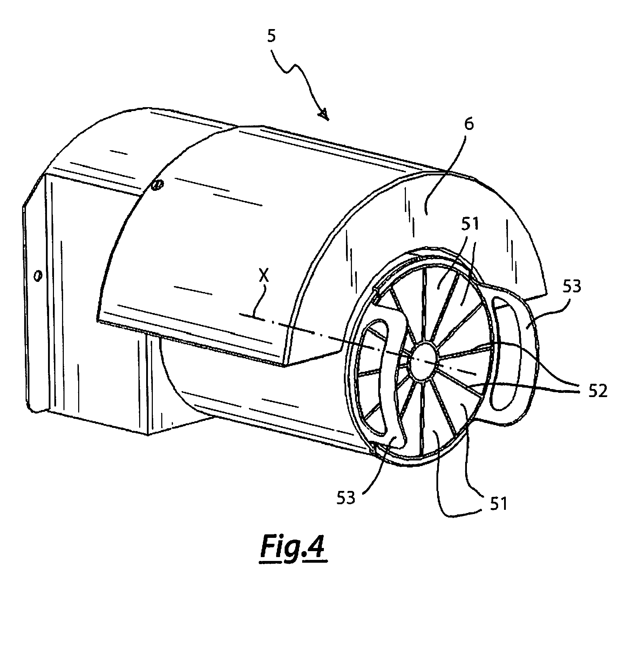

The properties and advantages of such equipment will be clearer and more evident from the following description, with reference to the annexed figures, showing in particular:
● Figure 1 is a schematic perspective view of the equipment according to the present invention;
● Figure 2 is a front schematic view of the equipment in a first operative condition according to the present invention;
● Figure 3 is a front schematic view of the equipment in a second operative condition according to the present invention;
● Figure 4 is a perspective schematic view of the drum of the equipment according to the present invention. With reference to the cited figures, the equipment according to the present invention comprises an irradiation chamber 3 inside which the biological products to irradiate can be positioned, and at least an irradiation or radiating source 4 positioned near said chamber.

The irradiation source (comprising any source of ionizing radiations, which can indifferently be a gamma-ray source or a X-ray tube) and the chamber can be moved one with respect to the other, so to obtain a focusing condition of the radiations, which maximizes their intensity with a consequent reduction of the treatment time, by permitting at the same time to modify the irradiated volume in order to be similar to the dimensions of the object to be treated.

Such movement permits the intersection of said beam with a predetermined volume of the chamber, according to the relative distance between them. In particular, the intersection of radiation cone F generated by the beam, for example with the base of the chamber, can have a greater or smaller diameter, according to the distance between the source of the beams and the chamber itself.

This determines a regulation of the intensity of the radiations in the chamber, which can be used for optimizing the exposition of the biological materials. Advantageously, the movement of the source and/or of the chamber is made by means of electric motors, controlled by a central electronic processing unit of the equipment. According to a feature of the present invention, inside the irradiation chamber an extractable drum 5 can be inserted, provided with a plurality of compartments 51. Advantageously, said drum 5 is adapted to rotate on itself in the chamber, in order to permit a uniform exposition of all compartments 51. Furthermore, according to another feature of the present invention, a reflecting diffuser screen 6 of the radiation emitted by the source is positioned behind the drum, on the opposite side of the one directly irradiated, in order to let the radiations return on the drum, for further irradiating it, by increasing in such a way its dose and consequently obtaining a reduction of the treatment time.

Advantageously, the cylindrical drum is placed in the chamber with its longitudinal axis X perpendicular to the direction of incidence of the radiation beam. The rotation of the drum is in fact possible around its longitudinal axis.

Furthermore, the drum is provided with handles 53, adapted to facilitate its insertion and extraction from the chamber. Obviously in the chamber coupling means of the drum are provided on a rotary shaft or hub, in order to permit its rotation. Such coupling means are for example electromagnetic means.

Such rotation of the drum permits a uniform exposition of all compartments 51 to the radiations, in order to expose their contents in a homogeneous way to a radiation beam, which is perpendicular to the rotary axis itself, by facilitating at the same time the mixing of the substance irradiated, when in a liquid state.

The drum is preferably used when the biological material to irradiate is made of sacs of blood, which can be easily positioned in the compartments.

The equipment according to the present invention has, thanks to the rear diffuser and to the possibility of approaching, when needed, the source of the beams to the treated object, the main benefit to increase the radiation dose emitted, with a consequent important reduction of the treatment times (which is fundamental in case of treatment of sacs of blood); furthermore the possibility of using a drum which rotates the sacs of cylindrical shape, optimizes the homogeneity of the dose, and avoids the aggregation of platelets. Another important property is the possibility of choosing the treatment with the use of a drum, which is inserted in the chamber as in figure 2) or in a general treatment without the drum in the chamber (as in figure 3) or furthermore without the entire module for rotating the drum if it is necessary to increase at the maximum the volume of the irradiation chamber. Such a difference is based on the fact that the source of the beams is mounted on a motorized structure which permits a focusing of the beams on the drum, in case of treatment of blood and in the general case on a greater area.

## Claims

1. Equipment for the treatment of materials via irradiation with ionizing radiation comprising an irradiation chamber (3) inside which the products to be irradiated can be positioned and in which an extractable drum (5) provided with a plurality of compartments (51) for containers of material to be irradiated can be positioned,
**characterized in that**
said drum (5) is adapted to turn on itself in the chamber so as to enable a uniform exposure of all the compartments (51) to the radiation, said rotation of the drum occurring substantially about its longitudinal axis in order to expose its contents in a homogeneous way to a beam of radiation that is perpendicular to the axis of rotation itself favouring at the same time mixing of the irradiated substance in the case where it is in the liquid form.

2. Equipment according to claim 1, wherein positioned in the proximity of the drum but on the opposite side to the incident radiating beam is a reflecting diffuser screen (6) that enables the radiation to return on the drum to irradiate it further with the consequent recovery and enhancement of the radiation itself.

3. Equipment according to claim 2, wherein the source of irradiation and the chamber can be moved with respect to one another so as to obtain a condition of focusing of the radiation that maximizes the intensity thereof with consequent reduction of the treatment time, at the same time enabling modification of the volume irradiated by adapting it to the dimensions of the object to be treated.
